# EUROPEAN PATENT APPLICATION

(11) **EP 4 212 549 A1**
(43) Date of publication of application: **19.07.2023**
(21) Application number: 21831586.9
(22) Date of filing: 16.03.2021
(51) Int. Cl.: C07K 16/28, A61K 39/395, A61P 35/00

(54) **ANTI-GITR MONOCLONAL ANTIBODY AND MEDICAL USE THEREOF**

(30) Priority: 28.06.2020 CN 202010597133
(71) Applicant: Dongda Bioscience Inc. (Suzhou), Taicang, Jiangsu 215400 (CN)
(72) Inventor: QIU, Junzhuan, Jiangsu 215400 (CN); SUN, Ziyong, Jiangsu 215400 (CN); WANG, Zhensheng, Jiangsu 215400 (CN); SUN, Kai, Jiangsu 215400 (CN); ZHOU, Man, Jiangsu 215400 (CN); CHEN, Junyong, Jiangsu 215400 (CN); SUN, Jian, Jiangsu 215400 (CN); OU, Rishan, Jiangsu 215400 (CN)
(74) Representative: Boco IP Oy Ab
(86) International application number: PCT/CN2021/080981
(87) International publication number: WO 2022/001189

(57) **Abstract**

Provided are an anti-GITR monoclonal antibody and a humanized antibody thereof, which can be used to prepare a drug used for activating immune response, particularly a related drug for treating cancer.

## Description

### TECHNICAL FIELD

The present disclosure pertains to the field of tumor and immunology drugs, and in particular, relates to an anti-GITR monoclonal antibody and medical use thereof.

### BACKGROUND

GITR is a glucocorticoid-induced TNFR-related protein, also known as TNFRSF18 or CD357, and is a type I transmembrane protein highly expressed in Treg cells and lowly expressed in naive and memory T cells [Shimizu et al., (2002), Nat. Immunol. 3:135-42; Nocentini et al., (2009), Adv. Exp. Med. Biol. 647:156-73; McHughet et al., (2002), Immunity 16:311-23; Nocentini et al., (2005), Eur J Immunol 35:1016-1022]. After being activated, naive T cells and Treg cells rapidly up-regulate the expression of GITR (Shimizu et al., (2002), Nat. Immunol. 3:135-42; Krausz et al., (2007, Scientific World Journal 7:533-66)). There is also low to moderate expression of GITR in activated innate immune cells such as natural killer cells, macrophages and dendritic cells (DCs) [Clouthier et al., (2014), Growth Factor Rev. 25:91-106]. GITR ligands (GITRLs) are type II transmembrane proteins belonging to the TNF superfamily, a certain amount of which are found expressed on the surfaces of endothelial cells including B cells, macrophages and dendritic cells (DCs)), as well as activated antigen-presenting cells (APCs) [Yu et al., (2003), Biochem. Biophys. Res. Commun.310: 433-438; Toneet et al., (2003), Proc. Natl. Acad. Sci. USA 100: 15059-15064].

Through the interaction between GITRL or GITR agonist antibodies and GITR, GITR can be stimulated to enhance TCR-induced T cell proliferation and cytokine production [Ronchetti et al., (2004), Eur. J. Immunol. 34:613-622; Tone et al., (2003), Proc. Natl. Acad. Sci. USA 100: 15059-15064; Stephens et al., (2004), J. Immunol. 173: 5008-5020], and reduce sensitivity of effector T cells to Treg cell-mediated immunosuppression [Ronchetti et al., (2007), Eur. J. Immunol. 37:1165-1169; Stephens et al., (2004), J. Immunol. 173:5008-5020]. Activation of GITR may recruit TNFR-associated factors 2 (TRAF 2) and 5 (TRAF5), and induce activation of transcription factor NF-κB and MAP kinase-induced signaling pathways [Snellet et al., (2011), Immunol. Rev. 244: 197-217; Snell et al., (2010), J. Immunol. 185: 7223-34], and induce transcription of anti-apoptotic Bcl-XL proteins, thereby reducing T cell apoptosis caused by cell activation [Ronchetti et al., (2004), Eur. J. Immunol. 34:613-622; Esparza et al., (2006), J. Biol. Chem. 281:8555-64]. The activation of GITR can further lead to activation of NFkB-dependent TRAF6 and production of IL-9, thereby enhancing the function of DC cells and activating the cytotoxic response of T lymphocytes [Kim et al., (2015), Nat. Med. 21: 1010-7]. GITR signaling can also promote degradation of FoxP3 [Cohenet et al., (2010), PLoS ONE 5:10436; Schaer et al., (2013), Cancer Immunology Research 1:320-331] and phosphorylation of c-Jun N-terminal kinase (JNK) [Joetham et al., (2012), J Biol. Chem. 287: 17100-17108], thereby further inhibiting the immune suppression function of Treg cells. In addition, it has also been reported that anti-GITR antibodies can reverse the inhibitory effect of Treg cells on B cell-mediated antibody production [Cava et al., (2004), J. Immunol. 173:3542-3548].

Preclinical studies have shown that activation of the GITR signaling pathway can significantly enhance elimination of tumors. The effect depends on activation of immune effector cells [Ramirez et al., (2006), J Immunol 176:6434-6442; Boczkowski et al., (2009), Cancer Gene Ther. 16:900-911; Mitsui et al., (2010), Clin. Cancer Res. 16:2781-91], and also depends on removal or inhibition of the immunosuppression activity of Treg cells [Cohen et al., (2010), PLoS ONE 5: e10436; Coe et al., (2010), Cancer Immunol. Immunother. 59:1367-77; Xiao et al., (2015), Nat. Commun. 6:8266]. When GITR activating antibodies are used alone, fewer Treg cells may enter tumor tissues, and foxp3 is not expressed in Treg cells in the tumor, thereby locally eliminating the immunosuppressive effect of Treg cells in the tumor. Ultimately, the Teff/Treg cell ratio is increased in the tumor microenvironment, and the activity and function of Teff cells are enhanced (Cohen et al., (2010), PLoS ONE 5: e10436). In an ovarian cancer model, the combination of anti-GITR and anti-PD-1 antibodies could improve patient survival overall; and after 90 days of treatment, up to 20% of mice were tumor-free. After analyses of the results of leukocytes (TILs) entering tumors in mice, it was found that the number of CD4+ and CD8+ effector memory T cells was significantly increased, while the number of Treg immune regulatory cells was significantly reduced [Lu et al., (2014), J . Transl. Med. 12:36-47]. Although monotherapy was not significantly effective with GITR antibodies, their combination with anti-CTLA4 antibodies showed a significantly enhanced synergistic antitumor effect in MethA and CT26 tumor models [Ko et al., (2005), J. Exp. Med. 202:885-91; Mitsui et al., (2010), Clin. Cancer Res. 16:2781-91].

### SUMMARY

In view of this, in the present disclosure, an anti-GITR monoclonal antibody, which has excellent effect of activating the activities of GITR and immune cell and blocking the interaction between GITR-L and GITR, is obtained through hybridoma technology; and the antibody is successfully humanized. The antibody has prominent application potential in the preparation of medications for stimulating and improving immune responses as well as medications for treating cancer.

The present disclosure provides an anti-GITR monoclonal antibody or an antigen-binding fragment thereof, which comprises a heavy chain and a light chain, where an amino acid sequence of CDR1 of the heavy chain is selected from the group consisting of SEQ ID NOs: 2, 10, 18, 26, 34, 42, 50, 58, 66, 74, 82, 90, 98 and 106; an amino acid sequence of CDR2 of the heavy chain is selected from the group consisting of SEQ ID NOs: 3, 11, 19, 27, 35, 43, 51, 59, 67, 75, 83, 91, 99 and 107; an amino acid sequence of CDR3 of the heavy chain is selected from the group consisting of SEQ ID NOs: 4, 12, 20, 28, 36, 44, 52, 60, 68, 76, 84, 92, 100 and 108; an amino acid sequence of CDR1 of the light chain is selected from the group consisting of SEQ ID NOs: 6, 14, 22, 30, 38, 46, 54, 62, 70, 78, 86, 94, 102 and 110; an amino acid sequence of CDR2 of the light chain is selected from the group consisting of SEQ ID NOs: 7, 15, 23, 31, 39, 47, 55, 63, 71, 79, 87, 95, 103 and 111; and an amino acid sequence of CDR3 of the light chain is selected from the group consisting of SEQ ID NOs: 8, 16, 24, 32, 40, 48, 56, 64, 72, 80, 88, 96, 104 and 112, and wherein the heavy and light chains of the antigen-binding fragment comprise amino acid sequences spanning from CDR1 through CDR3 of the heavy and light chains of the antibody, respectively.

Further, in the anti-GITR monoclonal antibody or the antigen-binding fragment thereof according to the present disclosure, an amino acid sequence of a heavy chain variable region is selected from the group consisting of SEQ ID NOs: 1, 9, 17, 25, 33, 41, 49, 57, 65, 73, 81, 89, 97 and 105, and an amino acid sequence of a light chain variable region is selected from the group consisting of SEQ ID NOs: 5, 13, 21, 29, 37, 45, 53, 61, 69, 77, 85, 93, 101 and 109.

Further, the heavy chain and the light chain of the anti-GITR monoclonal antibody or the antigen-binding fragment thereof according to the present disclosure are humanized; and an amino acid sequence of a humanized heavy chain variable region is selected from the group consisting of SEQ ID NOs: 113, 115, 117 and 119; and an amino acid sequence of a humanized light chain variable region is selected from the group consisting of SEQ ID NOs: 114, 116, 118 and 120.

Further, in the humanized anti-GITR monoclonal antibody or antigen-binding fragment thereof according to the present disclosure, a full-length amino acid sequence of the humanized heavy chain is selected from the group consisting of SEQ ID NOs: 121, 123, 125, 127, 128, 129 and 130, and a full-length amino acid sequence of the humanized light chain is selected from the group consisting of SEQ ID NOs: 122, 124, 126 and 131.

Further, the present disclosure correspondingly discloses a nucleotide sequence encoding the above anti-GITR monoclonal antibody or the antigen-binding fragment thereof.

Further, the present disclosure discloses the above anti-GITR monoclonal antibody or the antigen-binding fragment thereof for use in preparation of: a medication for activating T lymphocytes, a medication for blocking binding of GITR-L to GITR, or a medication for increasing expression of IL-2 and IFN-γ in T lymphocytes.

Further, the present disclosure discloses a monoclonal antibody conjugate, comprising a monoclonal antibody and a conjugating part, where the monoclonal antibody is the anti-GITR monoclonal antibody or the antigen-binding fragment thereof according to the present disclosure, and the conjugating part is selected from one or more of a radionuclide, a medication, a toxin, a cytokine, a cytokine receptor fragment, an enzyme, fluorescein, and biotin.

Further, the present disclosure discloses the above monoclonal antibody conjugate for use in preparation of: a medication for activating T lymphocytes, a medication for blocking binding of GITR-L to GITR, or a medication for increasing expression of IL-2 and IFN-γ in T lymphocytes.

Further, the present disclosure discloses the above monoclonal antibody conjugate for use in preparation of a medication for preventing, treating, and/or assistantly treating a tumor.

The present disclosure has the following benefits: in the present disclosure, recombinant GITR prepared with a mammalian cell expression system is used as an antigen to immunize mice, and the spleen cells of the mice are fused with myeloma cells to obtain hybridoma cells. After multiple cloning and screening of a large number of the hybridoma cells, some monoclonal hybridoma cell lines are obtained. These hybridoma cell lines can produce monoclonal antibodies that specifically bind to GITR with high affinity, can promote the secretion of IL-2 and INF-γ in mixed lymphocyte reactions, and can effectively block the binding of GITR-L to GITR, and also can be used to construct humanized antibodies in a complementarity determinant grafting method by cloning genes encoding the light and heavy chain variable regions of the antibodies via RT-PCR. *In vitro* functional tests show that these humanized GITR antibodies can specifically bind to GITR proteins with high affinity, can significantly promote the secretion of cytokines IL-2 and INF-γ by T cells, and can block the binding of GITR-L to GITR. The test results show that the monoclonal antibody or the antigen-binding fragment thereof according to the present disclosure, or the monoclonal antibody conjugate comprising the monoclonal antibody or the antigen-binding fragment thereof according to the present disclosure has good application potential in preparation of medications for activating T lymphocytes, medications for increasing the expression of IL-2 and INF-γ in T lymphocytes, medications for blocking the binding of GITR-L to GITR, and medications for preventing, treating, and/or assistantly treating tumors.

### BRIEF DESCRIPTION OF THE DRAWINGS

FIG. 1 shows EC50 for the binding of the humanized anti-GITR antibodies to GITR-mFc proteins detected in ELISA assay;
FIG. 2 shows the result of the binding of the humanized GITR antibodies to GITR on a cell membrane in FACS assay;
FIG. 3 shows the blocking effect of the humanized GITR antibodies on the binding of GITR-L to Jurkat-GITR cells;
FIG. 4 shows the GITR activation activity of the humanized anti-GITR antibodies via 293T-GITR-NFkb-luc cell reporter gene assay;
FIG. 5 shows the result of the activation of Jurkat-GITR cells by the humanized GITR antibodies to release cytokine IL2 without cross-linking;
FIG. 6 shows the result of the activation of Jurkat-GITR cells by the humanized GITR antibodies to release cytokine IL2 with cross-linking;
FIG. 7 shows the effect of the humanized GITR antibodies on the production of cytokine IL2 by CD4 T cells; and
FIG. 8 shows the effect of the humanized GITR antibodies on the production of cytokine IFN-γ by CD4 T cells.

### DETAILED DESCRIPTION

Technical solutions of the present disclosure are further described in the following embodiments. Persons skilled in the art should understand that the examples are only intended to help understand the present disclosure, and should not be construed as specific limitations on the present disclosure; the assays used in the following examples are all conventional methods unless otherwise specified; and materials, reagents and the like used in the following examples are all commercially available unless otherwise specified. Tables 1 to 3 show the amino acids of the antibodies involved in the present disclosure.

**Table 1 Light Chain CDR Sequences of GITR Antibodies**

| Name | LCDR | SEQ ID NO | Sequence |
|---|---|---|---|
| 4E1 | 1 | 6 | KSSQSLLDSDGKTYLN |
| | 2 | 7 | LVSELDS |
| | 3 | 8 | WQGTHFPWT |
| 8H4 | 1 | 14 | SASSSVSYMY |
| | 2 | 15 | DTSTLAS |
| | 3 | 16 | QQWSSFPPT |
| 4E4 | 1 | 22 | RSSQSIVYSNGNTYLN |
| | 2 | 23 | KVSNRFS |
| | 3 | 24 | FQGSHVPWT |
| 3F5 | 1 | 30 | KSSQSLLDRDGKTYLN |
| | 2 | 31 | LVSILDS |
| | 3 | 32 | WQGTHFPWT |
| 4C1 | 1 | 38 | RASENIYSNLA |
| | 2 | 39 | AATNLAD |
| | 3 | 40 | QHFWGPPWT |
| 7D7A2 | 1 | 46 | TASSSVSSSYFH |
| | 2 | 47 | STSNLAS |
| | 3 | 48 | HQYHHSPRT |
| 7E11 | 1 | 54 | RASENIYSNLA |
| | 2 | 55 | AATNLAD |
| | 3 | 56 | QHFWGPPWT |
| 10E11 | 1 | 62 | RASQSVNNYLH |
| | 2 | 63 | YASQSIS |
| | 3 | 64 | QQSNRWPLT |
| 3G12 | 1 | 70 | RASENIYSNLA |
| | 2 | 71 | AATNLAD |
| | 3 | 72 | QHFWGPPWT |
| 4B11 | 1 | 78 | RASQSVSTSTYSYVH |
| | 2 | 79 | YASNLES |
| | 3 | 80 | QHSWGNPWT |
| 5F1 | 1 | 86 | SASSSVSYMH |
| | 2 | 87 | STSNLAS |
| | 3 | 88 | HQRSGHTFT |
| 5F6 | 1 | 94 | RASQSVSTSSYSYMH |
| | 2 | 95 | YASNLES |
| | 3 | 96 | QHGWEIPYT |
| 13G12 | 1 | 102 | GASSSVSYMY |
| | 2 | 103 | DTFNLAS |
| | 3 | 104 | QHWSSYPPT |
| 9B3 | 1 | 110 | RASQSVNNYLH |
| | 2 | 111 | YVSQSIS |
| | 3 | 112 | QQSNRWPLT |

**Table 2 Heavy Chain CDR Sequences of GITR Antibodies**

| Name | HCDR | SEQ ID NO | Sequence |
|---|---|---|---|
| 4E1 | 1 | 2 | SDYAWN |
| | 2 | 3 | YIRNSGTTSYNPSLKS |
| | 3 | 4 | SGHHDLFDY |
| 8H4 | 1 | 10 | DYYMN |
| | 2 | 11 | NINPNHDYTSYNQKFKG |
| | 3 | 12 | YYYAMDY |
| 4E4 | 1 | 18 | DHYMN |
| | 2 | 19 | DIDPKNGGTLYNQNFKG |
| | 3 | 20 | REAMDY |
| 3F5 | 1 | 26 | DHYMH |
| | 2 | 27 | DINPNSGGTINNQNFKG |
| | 3 | 28 | GFSGLAY |
| 4C1 | 1 | 34 | SYWMQ |
| | 2 | 35 | TIYPGDGDTRYTQKFKG |
| | 3 | 36 | FEGRQFAY |
| 7D7A2 | 1 | 42 | RYWIE |
| | 2 | 43 | EILPGSGYNNSNDKFKG |
| | 3 | 44 | KGTFYTMDH |
| 7E11 | 1 | 50 | SYWMQ |
| | 2 | 51 | TIYPGDGDTRYTQKFKG |
| | 3 | 52 | FEGRQFAY |
| 10E11 | 1 | 58 | RYWIE |
| | 2 | 59 | EILPGSDYANYNEKFKG |
| | 3 | 60 | KGAYYALDF |
| 3G12 | 1 | 66 | SYWMQ |
| | 2 | 67 | TIYPGDGDTRYTQKFKG |
| | 3 | 68 | FYGGAMDS |
| 4B11 | 1 | 74 | DSVLH |
| | 2 | 75 | YIHPYNDDTKFHDSFKG |
| | 3 | 76 | NDELRVALDY |
| 5F1 | 1 | 82 | SDYAWN |
| | 2 | 83 | YITYSGSTGYNPSLKS |
| | 3 | 84 | DWPFAY |
| 5F6 | 1 | 90 | RYTMS |
| | 2 | 91 | YISNGGGSTYYPDTVKG |
| | 3 | 92 | AEAGTDWFAY |
| 13G12 | 1 | 98 | SNWMN |
| | 2 | 99 | NIYPSNGGTNYNEKFKS |
| | 3 | 100 | HSNYVGAMDY |
| 9B3 | 1 | 106 | RYWIE |
| | 2 | 107 | EILPGSDYANYNEKFKG |
| | 3 | 108 | KGAYYALDF |

**Table 3 Light Chain and Heavy Chain Variable Region Sequences and Full-length Antibody Sequences of Antibodies**

| Name | Region¹ | SEQ ID NO | Sequence |
|---|---|---|---|
| 4E] (murine) | Light chain variable | 5 | DVVMTQTPLTLSFTIGQPASISCKSSQSLLDSDGKTYLNWLLQR PGQSPKRLIYLVSELDSGVPDRFTGSGSGTDFTLQISRVEAEDLG VYYCWQGTHFPWTFGGGTKLEIKRA |
| 4E1 (murine) | Heavy chain variable | 1 | DVQLQESGPGLVKPSQSLSLTCTVTGYSITSDYAWNWIRQFPG NKLEWMGYIRNSGTTSYNPSLKSRISITRDTSKNHFFLQLNSVT TEDTATYYCTRSGHHDLFDYWGQGTTLTVSS |
| RH4 (murine) | Light chain variable | 13 | QIVLTQSPAIMSAFPGEKVTMTCSASSSVSYMYWYQQKPGSSP RLLIFDTSTLASGVPVRFSGSGSGTSHSLTISRMEAEDAATYYCQ QWSSFPPTFGAGTRLELKRA |
| RH4 (murine) | Heavy chain variable | 9 | EVQLQQSGPELVRPGASVNISCKASGYTFTDYYMNWVKQSHG KSLEWIGNINPNHDYTSYNQKFKGKATLTVDKSSNTAYMELRS LTSEDSAVYYCARYYYAMDYWGQGTSVTVSS |
| 4E4 (murine) | Light chain variable | 21 | DVLMTQIPLSLPVSFGDQASISCRSSQSIVYSNGNTYLNWYLQK PGQSPKLLIYKVSNRFSGVPDRFSGSGSGTDFTLKISRVEAEDLG VYYCFQGSHVPWTFGGGTKLEIKRA |
| 4E4 (murine) | Heavy chain variable | 17 | EVQLQQSGPELVKPGASVKISCKASGYTFTDHYMNWVKQSHG KSLEWIGDIDPKNGGTLYNQNFKGRATLTVDKSSSTAYMDLRS LTSEDSAVYYCARREAMDYWGQGTSVTVSS |
| 3F5 (murine) | Light chain variable | 29 | DVVMTQTPLTLSVTIGQPASISCKSSQSLLDRDGKTYLNWLFQR PGQSLKRLIYLVSILDSGVPDRFTGSGSGTDFTLKISRVEAGDLG IYYCWQGTHFPWTFGGGTKLEIKRA |
| 3F5 (murine) | Heavy chain variable | 25 | EVQLQQSGPELVKPGTSVKISCKASGYTFTDHYMHWVRQSHG KSLEWIGDINPNSGGTINNQNFKGKATLTVDKSSNTAYMGLHS LTSEDSAVYYCASGFSGLAYWGQGTLVTVSA |
| 4C1 (murine) | Light chain variable | 37 | MTQSPASLSVSVGETVTITCRASENIYSNLAWYQQKQGKSPQL LVYAATNLADGVPSRFSGSGSGTQYSLKINSLQSEDFGSYYCQ HFWGPPWTFGGGTKLEIKRA |
| 4C1 murine | Heavy chain variable | 33 | QVQLQQSGAELARPGASVKLSCKASGYTFTSYWMQWVKQRP GQGLEWIGTIYPGDGDTRYTQKFKGKATLTADKSSNTAYMQL SSLASEDSAVYYCARFEGRQFAYWGQGTLVTVSA |
| 7D7 (murine) | Light chain variable | 45 | DIVMTQSTAIMSASLGERVTMTCTASSSVSSSYFHWYQQKPGS SPKLWIYSTSNLASGVPARFSGRGSGTSYSLTISSVEAEDAATY YCHQYHHSPRTFGGGSKLEIKRA |
| 7D7 (murine) | Heavy chain variable | 41 | QVQLQQSGAEVMKPGASVKISCKATDYIFSRYWIEWVKQRPG HGLEWIGEILPGSGYNNSNDKFKGKATFTADTSSNTAYMQLSS LTFEDSAVYYCARKGTFYTMDHWGQGTSVTVSS |
| 7E11 (murine) | Light chain variable | 53 | DIQMTQSPASLSVSVGETVTITCRASENIYSNLAWYQQKQGKSP QLLVYAATNLADGVPSRFSGSGSGTQYSLKINSLQSEDFGSYYC QHFWGPPWTFGGGTKLEIKRA |
| 7E11 (murine) ) | Heavy chain variable | 49 | QVQLQQSGAELARPGASVKLSCKASGYTFTSYWMQWIKQRPG QGLEWIGTIYPGDGDTRYTQKFKGKATLTADKSSSTAYMQLSS LASEDSAVYYCARFEGRQFAYWGQGTLVTVSA |
| 10E11 (murine) | Light chain variable | 61 | DIVLTQSPATLSVTPGERVSLSCRASQSVNNYLHWYQQKSHESP RLLISYASQSISGISSRFSGSGSGTDFTLHHSVETEDFGMYFCQQS NRWPLTFGAGTKLELKRA |
| WEll (murine) | I leavy chain variable | 57 | EVKLEESGGGLVNLGASVKISCKATGYPVSRYWIEWLKQRPGII GPEWIGEILPGSDYANYNEKFKGKATFTADISSNSAYMQLSSLT SEDSAVYYCSRKGAYYALDFWGQGTSVTVSS |
| 3G12 (murine) | Light chain variable | 69 | DIQMTQSPASLSVSVGETVTITCRASENIYSNLAWYQHKQGKSP QLLVYAATNLADGVPSRFSGGGSGTQYSLKINSLQSEDFGTYY CQHFWGPPWTFGGGTKLEIKRA |
| 3G12 (murine) | Heavy chain variable | 65 | QVQLQQSGAELARPGASVKLSCKASGYTFTSYWMQWVKQRP GQGLEWIGTIYPGDGDTRYTQKFKGKATLTADKSSSTVYMQLT SLASEDSAVYYCARFYGGAMDSWGQGTSVTVSS |
| 4B11 (murine) | Light chain variable | 77 | DIVLTQSPASLAVSLGQRATISCRASQSVSTSTYSYVHWYQQKP GQPPKLLIKYASNLESGVPARFSGRGSGTDFTLSIHPVEGEDAA TYYCQHSWGNPWTFGGGTKLEIKRA |
| 4B11 (murine) | Heavy chain variable | 73 | EVQLQQSGPELVKPGASVKMSCKASGFTFTDSVLHWVKQKPG QGLEWIGYIHPYNDDTKFHDSFKGKATLTSDKSSTTAYMELSS LTSEDSAVYYCSRNDELRVALDYWGQGTSVTVSS |
| 5F1 (murine | Light chain variable | 85 | QIVLTQSPAIMSASPGEKATITCSASSSVSYMHWFQQRPGTSPK LWIYSTSNLASGVPARFSGSGSGTSFSLTISRMEAEDAATYYCH QRSGHTFTFGSGTKLEIKRA |
| 5F1 murine) | Heavy chain variable | 81 | DVQLQESGPGLVKPSHSLSLTCTVTGYSITSDYAWNWIRQFPG NNLEWMGYITYSGSTGYNPSLKSRISITRDTSKNQFFLQLNSVT AEDTATYYCARDWPFAYWGQGTLVTVSA |
| 5F6 | Light chain | 93 | DIVLTQSPASLAVSLGQRATISCRASQSVSTSSYSYMHWYQQKP GQPPKLLIKYASNLESGVPARFSGSGSGTDFTLNIHPVEEEDTAT |
| (murine) | variable | | YYCQHGWEIPYTFGGGTKLEIKRA |
| 5F6 (murine) | Heavy chain variable | 89 | EVKLEESGGGLVQPGGSLKLSCAASGFTLSRYTMSWVRQTPEK RLEWVAYISNGGGSTYYPDTVKGRFTTSRDNAKNTLYLQMSSL KSEDTAMYYCGRAEAGTDWFAYWGQGTLVTVSA |
| 13G12 (murine) | Light chain variable | 101 | QIVLTQSPAIMSASPGEKVTMTCGASSSVSYMYWFQQKPGSSP RLLIYDTFNLASGVPVRFSGSESGTSYSLTISRMEAEDAATYYC QHWSSYPPTFGSGTKLEIDRA |
| 13G12 (murine) | Heavy chain variable | 97 | QVQLQQPGTELVKPGASVKLSCKASGYTFTSNWMNWVKQRP GHGLEWIGNIYPSNGGTNYNEKFKSKATLTVDKSSSTAYMQLR GLTSEDSAVYYCVRHSNYVGAMDYWGQGTSVTVSS |
| 9B3 (murine) | Light chain variable | 109 | DIVMTQSPATLSVTPGERVSLSCRASQSVNNYLHWYQQKSHES PRLLISYVSQSISGISSRFSGSGSGTDFTLIIHSVETEDFGMYFCQQ SNRWPLTFGAGTKLELK |
| 9B3 (murine) | Heavy chain variable | 105 | QVQLQQSGAELMNLGASVKISCKATGYPVSRYWIEWLKQRPG HGPEWIGEILPGSDYANYNEKFKGKATFTADISSNSAYMQLSSL TSEDSAVYYCSRKGAYYALDFWGQGTSVTVSS |
| 4F.4 (humanized) | Heavy chain variable | 113 | QVQLVQSGAEVKKPGASVKVSCKASGYTFTDHYMNWVRQAP GQGLEWIGDIDPKNGGTLYNQNFKGRATLTVDKSISTAYMELS RLRSDDTAVYYCARREAMDYWGQGTLVTVSS |
| 4E4 (humanized) d) | Light chain variable | 114 | DVVMTQSPLSLPVTPGEPASISCRSSQSIVYSNGNTYLNWYLQK PGQSPQLLIYKVSNRFSGVPDRFSGSGSGTDFTLKISRVEAEDVG VYYCFQGSHVPWTFGGGTKVEIK |
| 4B11 (humanized) | Heavy chain variable | 115 | EVQLVQSGAEVKKPGASVKVSCKASGFTFTDSVLIIWVRQAPG QRLEWIGYIHPYNDDTKFHDSFKGRATLTSDKSASTAYMELSS LRSEDTAVYYCSRNDELRVALDYWGQGTLVTVSS |
| 4B11 (humanized) | Light chain variable | 116 | EIVLTQSPATLSLSPGERATLSCRASQSVSTSTYSYVHWYQQKP GQAPRLLIKYASNLESGIPARFSGSGSGTDFTLTISSLEPEDFAVY YCQHSWGNPWTFGGGTKVEIK |
| 3F5 (humanized) | Heavy chain variable | 117 | QVQLVQSGAEVKKPGASVKVSCKASGYTFTDHYMHWVRQAP GQGLEWIGDINPNSGGTINNQNFKGRATLTVDKSISTAYMELSR LRSDDTAVYYCARGFSGLAYWGQGTLVTVSS |
| 3F5 (humanized) | Light chain variable | 118 | DIVMTQTPLSSPVTLGQPASISCKSSQSLLDRDGKTYLNWLQQR PGQPPRRLIYLVSILDSGVPDRFSGSGAGTDFTLKISRVEAEDVG VYYCWQGTHFPWTFGQGTKLEIK |
| 10E11 (humanize d) | heavy chain variable | 119 | EVQLVQSGAEVKKPGESLRISCKGSGYPVSRYWIEWVRQMPG KGLEWIGEILPGSDYANYNEKFKGHATFSADISISTAYLQWSSL KASDTAMYYCSRKGAYYALDFWGQGTLVTVSS |
| 10E11 (humanized) | light chain variable | 120 | EIVMTQSPATLSVSPGERATLSCRASQSVNNYLHWYQQKPGQA PRLLISYASQSISGIPARFSGSGSGTEFTLTISSLQSEDFAVYYCQ QSNRWPLTFGQGTKLEIK |
| 4E4 (humanized) | Full heavy chain | 121 | QVQLVQSGAEVKKPGASVKVSCKASGYTFTDHYMNWVRQAP GQGLEWIGDIDPKNGGTLYNQNFKGRATLTVDKSISTAYMELS RLRSDDTAVYYCARREAMDYWGQGTLVTVSSASTKGPSVFPL APSSKSTSGGTAALGCLVKDYFPEPVTVSWNSGALTSGVHTFP AVLQSSGLYSLSSVVTVPSSSLGTQTYICNVNHKPSNTKVDKRV EPKSCDKTHTCPPCPAPELLGGPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHE ALHNHYTQKSLSLSPGK |
| 4E4 (humanized) | Full light chain | 122 | DVVMTQSPLSLPVTPGEPASISCRSSQSIVYSNGNTYLNWYLQK PGQSPQLLIYKVSNRFSGVPDRFSGSGSGTDFTLKISRVEAEDVG VYYCFQGSHVPWTFGGGTKVEIKRTVAAPSVFIFPPSDEQLKSG TASVVCLLNNFYPREAKVQWKVDNALQSGNSQESVTEQDSKD STYSLSSTLTLSKADYEKHKVYACEVTHQGLSSPVTKSFNRGEC |
| 4B11 (humanized) | Full heavy chain | 123 | EVQLVQSGAEVKKPGASVKVSCKASGFTFTDSVLIIWVRQAPG QRLEWIGYIHPYNDDTKFHDSFKGRATLTSDKSASTAYMELSS LRSEDTAVYYCSRNDELRVALDYWGQGTLVTVSSASTKGPSVF PLAPSSKSTSGGTAALGCLVKDYFPEPVTVSWNSGALTSGVHT FPAVLQSSGLYSLSSVVTVPSSSLGTQTYICNVNIIKPSNTKVDK RVEPKSCDKTHTCPPCPAPELLGGPSVFLFPPKPKDTLMISRTPE VTCVVVDVSHEDPEVKFNWYVDGVEVHNAKTKPREEQYNST YRVVSVLTVLHQDWLNGKEYKCKVSNKALPAPIEKTISKAKG QPREPQVYTLPPSRDELTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 4B11 (humanized) | Full light chain | 124 | EIVLTQSPATLSLSPGERATLSCRASQSVSTSTYSYVHWYQQKP GQAPRLLIKYASNLESGIPARFSGSGSGTDFTLTISSLEPEDFAVY YCQHSWGNPWTFGGGTKVEIKRTVAAPSVFIFPPSDEQLKSGT ASVVCLLNNFYPREAKVQWKVDNALQSGNSQESVTEQDSKDS TYSLSSTLTLSKADYEKHKVYACEVTHQGLSSPVTKSFNRGEC |
| 3F5 (humanized) -hIgG1 | Full heavy chain | 125 | QVQLVQSGAEVKKPGASVKVSCKASGYTFTDHYMHWVRQAP GQGLEWIGDINPNSGGTINNQNFKGRATLTVDKSISTAYMELSR LRSDDTAVYYCARGFSGLAYWGQGTLVTVSSASTKGPSVFPLA PCSRSTSESTAALGCLVKDYFPEPVTVSWNSGALTSGVHTFPAV LQSSGLYSLSSVVTVPSSSLGTKTYTCNVDHKPSNTKVDKRVES KYGPPCPPCPAPEFLGGPSVFLFPPKPKDTLMISRTPEVTCVVVD VSQEDPEVQFNWYVDGVEVHNAKTKPREEQFNSTYRVVSVLT VLHQDWLNGKEYKCKVSNKGLPSSIEKTISKAKGQPREPQVYT LPPSQEEMTKNQVSLTCLVKGFYPSDIAVEWESNGQPENNYKT TPPVLDSDGSFFLYSRLTVDKSRWQEGNVFSCSVMHEALHNHY |
| | | | TQKSLSLSLG |
| 3F5 (humanized) -hIgG1 | Full light chain | 126 | DIVMTQTPLSSPVTLGQPASISCKSSQSLLDRDGKTYLNWLQQR PGQPPRRLIYLVSILDSGVPDRFSGSGAGTDFTLKISRVEAEDVG VYYCWQGTHFPWTFGQGTKLEIKRTVAAPSVFIFPPSDEQLKSG TASVVCLLNNFYPREAKVQWKVDNALQSGNSQESVTEQDSKD STYSLSSTLTLSKADYEKHKVYACEVTHQGLSSPVTKSFNRGEC |
| 10E11 (humanized) -hIgG1 | Full heavy chain | 127 | EVQLVQSGAEVKKPGESLRISCKGSGYPVSRYWIEWVRQMPG KGLEWIGEILPGSDYANYNEKFKGHATFSADISISTAYLQWSSL KASDTAMYYCSRKGAYYALDFWGQGTLVTVSSASTKGPSVFP LAPSSKSTSGGTAALGCLVKDYFPEPVTVSWNSGALTSGVHTF PAVLQSSGLYSLSSVVTVPSSSLGTQTYICNVNHKPSNTKVDKR VEPKSCDKTHTCPPCPAPELLGGPSVFLFPPKPKDTLMISRTPEV TCVVVDVSHEDPEVKFNWYVDGVEVHNAKTKPREEQYNSTY RVVSVLTVLHQDWLNGKEYKCKVSNKALPAPIEKTISKAKGQP REPQVYTLPPSRDELTKNQVSLTCLVKGFYPSDIAVEWESNGQP ENNYKTTPPVLDSDGSFFLYSKLTVDKSRWQQGNVFSCSVMHE ALHNHYTQKSLSLSPGK |
| 10E11V H(G24A/ A67V)-h IgG1 [10E11V H(AV)-h IgG1\| | Full heavy chain | 128 | EVQLVQSGAEVKKPGESLRISCKASGYPVSRYWIEWVRQMPG KGLEWIGEILPGSDYANYNEKFKGHVTFSADISISTAYLQWSSL KASDTAMYYCSRKGAYYALDFWGQGTLVTVSSASTKGPSVFP LAPSSKSTSGGTAALGCLVKDYFPEPVTVSWNSGALTSGVHTF PAVLQSSGLYSLSSVVTVPSSSLGTQTYICNVNHKPSNTKVDKR VEPKSCDKTHTCPPCPAPELLGGPSVFLFPPKPKDTLMISRTPEV TCVVVDVSHEDPEVKFNWYVDGVEVHNAKTKPREEQYNSTY RVVSVLTVLHQDWLNGKEYKCKVSNKALPAPIEKTISKAKGQP REPQVYTLPPSRDELTKNQVSLTCLVKGFYPSDIAVEWESNGQP ENNYKTTPPVLDSDGSFFLYSKLTVDKSRWQQGNVFSCSVMHE ALHNHYTQKSLSLSPGK |
| 10E11V H(G24A/ A67V)-h IgG1(3A ) [10E11V H(AV)-h IgG1(3A)] | Full heavy chain | 129 | EVQLVQSGAEVKKPGESLRISCKASGYPVSRYWIEWVRQMPG KGLEWIGEILPGSDYANYNEKFKGHVTFSADISISTAYLQWSSL KASDTAMYYCSRKGAYYALDFWGQGTLVTVSSASTKGPSVFP LAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPEV TCVVVDVSHEDPEVKFNWYVDGVEVHNAKTKPREEQYNATY RVVSVLTVLHQDWLNGKEYKCKVSNKALPAPIAATISKAKGQP REPQVYTLPPSRDELTKNQVSLTCLVKGFYPSDIAVEWESNGQP ENNYKTTPPVLDSDGSFFLYSKLTVDKSRWQQGNVFSCSVMHE ALHNHYTQKSLSLSPG |
| 10E11V H(G24A/ A67V) | Full heavy chain | 130 | EVQLVQSGAEVKKPGESLRISCKASGYPVSRYWIEWVRQMPG KGLEWIGEILPGSDYANYNEKFKGHVTFSADISISTAYLQWSSL KASDTAMYYCSRKGAYYALDFWGQGTLVTVSSASTKGPSVFP |
| hIgG4 [10E11V H(AV)-h IgG4] | | | LAPCSRSTSESTAALGCLVKDYFPEPVTVSWNSGALTSGVHTFP AVLQSSGLYSLSSVVTVPSSSLGTKTYTCNVDHKPSNTKVDKR VESKYGPPCPPCPAPEFLGGPSVFLFPPKPKDTLMISRTPEVTCV VVDVSQEDPEVQFNWYVDGVEVHNAKTKPREEQFNSTYRVVS VLTVLHQDWLNGKEYKCKVSNKGLPSSIEKTISKAKGQPREPQ VYTLPPSQEEMTKNQVSLTCLVKGFYPSDIAVEWESNGQPENN YKTTPPVLDSDGSFFLYSRLTVDKSRWQEGNVFSCSVMHEALH NHYTQKSLSLSLG |
| 10E11 humamzed | Full light chain | 131 | EIVMTQSPATLSVSPGERATLSCRASQSVNNYLHWYQQKPGQA PRLLISYASQSISGIPARFSGSGSGTEFTLTISSLQSEDFAVYYCQ QSNRWPLTFGQGTKLEIKRTVAAPSVFIFPPSDEQLKSGTASVV CLLNNFYPREAKVQWKVDNALQSGNSQESVTEQDSKDSTYSL SSTLTLSKADYEKHKVYACEVTHQGLSSPVTKSFNRGEC |
| | | | |

### Example 1: Production of Anti-GITR Hybridoma Antibodies

Purified human GITR (ECD)-mFc (Kim et al., 2007, Oncol Rep. 18: 1189) fusion proteins purchased from Dongda Bioscience were used as antigens, which were fully emulsified with complete Freund's adjuvant (Sigma, Cat No: F5581) of an equal volume, and used to subcutaneously immunize 6-8 week old Balb/c mice (purchased from Zhaoyan (Suzhou) New Drug Research Center Co., Ltd.) at a dose of 50 µg/mouse; and then the same dose of antigens were fully emulsified with incomplete Freund's adjuvant (Sigma, Cat No: F5506) every 2 weeks before being used to subcutaneously immunize the mice three times. After the three times of immunization, serum titers of the mice were measured, and the mice were intraperitoneally booster immunized 3 days before fusion. Using PEG Hybri-Max (Sigma, Cat No: 7181) as a fusion agent, spleen cells of the mice and SP2/0 cells were mixed at a ratio of 4:1. Fused cells were added into a 96-well plate (1×10⁵ cells/well), each well containing 0.1 mL of 1X HAT medium (Invitrogen, Cat No: 21060-017). 0.1 mL of HT (Invitrogen, Cat No: 11067-030) medium was added on day 3, the medium was pipetted out of the 96-well plate on day 7, and 0.2 mL of fresh HT medium was added. On day 9, a supernatant was collected for subsequent ELISA and FACS assays.

### Example 2: Screening and Analysis of Polyclonal Hybridomas Based on ELISA, FACS, and Reporter Gene Assays

These assays were intended to screen and select polyclonal hybridomas that can bind to GITR, block the interaction between GITR-L and GITR, and significantly activate the molecular activity of GITR, and polyclonal hybridomas with a high binding capability and high stimulation activity were selected for subclone to obtain required monoclonal hybridoma antibodies.

After the spleen cells of the immunized mice with high ELISA titers were fused with SP2/0 cells and cultured for 7 days, the hybridoma cells were first subjected to ELISA and FACS assays to determine and select positive hybridomas that could bind to GITR, and then 293T luciferase reporter gene assay was initially conducted to screen and select polyclonal hybridomas having functional responses. In the process, a total of more than forty polyclonal hybridoma antibodies with strong binding to GITR were identified. After the reporter gene assay, polyclonal hybridomas with significant binding and stimulation activities were selected for subsequent subclone screening. The hybridoma cells with strong binding to GITR were cryopreserved in liquid nitrogen.

ELISA assay: a 96-well ELISA plate (Corning, Cat No: 9018) was inoculated with GITR-ECD-hFc, left standing overnight at 4°C, and washed 3 times with washing buffer (PBS+0.05% Tween 20), then blocking buffer (PBS+1% BSA (Sigma, Cat No: V90093)) was added, and the 96-well ELISA plate was incubated for 1 hour and washed 3 times; hybridoma supernatant was added, the 96-well ELISA plate was incubated for 1 hour and washed 3 times, 100 µL of goat anti-mouse IgG secondary antibodies (Thermo, Cat No: 31432) diluted at a ratio of 1:10000 was added into each well, and the 96-well ELISA plate was incubated at room temperature for 1 hour and washed 3 times. 100 µL of TMB (Biosubstrate Technologies, Cat No: ES-002) was added into each well and left standing for 3 minutes for color development, then a stop solution (2N H₂SO₄) was added at a dose of 100 µL/well to terminate the reaction, and OD450×signal of each sample was measured with a Tecan Spark microplate reader.

FACS assay for the binding of the antibodies: 50 µL of the hybridoma supernatant tested positive in the foregoing ELISA assay was pipetted and mixed with 50 µL of 293T-GITR cells (1×10⁵ cells/well), and the mixture was added into a 96-well plate with a U-shaped bottom and incubated for 1 hour. After the mixture was centrifuged and washed with FACS buffer (PBS+3% FCS) twice, a solution of PE-labeled goat anti-mouse secondary antibodies (Biolegend, Cat No: 405307) diluted at a ratio of 1:400 was added, the mixture was incubated for 30 minutes, and washed with FACS buffer, and then the PE signals of 293T-GITR cells were detected by using BD C6 flow cytometer.

FACS assay for the blocking effect of the antibodies: FACS assay was conducted by using BD C6 to determine the blocking effect of the hybridoma antibodies on the binding of biotin-labeled human GITR ligands to Jurkat-hGITR cells. Jurkat-hGITR cells were suspended in FACS buffer (PBS containing 3% FBS). Diluted hybridoma antibodies were added into the cell suspension and the mixture was incubated in a 96-well plate for 1 hour at 4°C. Biotin-labeled human GITR ligand proteins were added into the wells of the 96-well plate and incubated for 1 hour at 4°C. After incubation, the 96-well plate was washed 3 times, and mouse anti-biotin PE antibodies (Biolgend, Cat No: 409004) were added and incubated at 4°C for 0.5 hours. The cells in the 96-well plate were washed 3 times and finally analyzed with BD C6.

Analysis of the function of the hybridoma anti-GITR antibodies in immune activation based on the reporter gene assay of Jurkat-GITR-NF-κB cells: FACS assay of reporter genes was conducted for Jurkat-GITR-NF-κB cell line with anti-GITR hybridoma supernatant. 25000 Jurkat-GITR-NF-κB cells were used in each reaction, these cells were added into each well of the 96-well culture plate, then 50 µL of hybridoma supernatant was further added into each well, and the 96-well culture plate was incubated for 4 hours at 37°C. Cells were lysed with 50 µL of Bright Glo reagent (Promega, Cat No: E2620), and signals were read under luminescent conditions with Tecan Spark.

### Example 3: Subclone of Polyclonal Hybridoma Cells, Amplification Culture and Antibody Purification of GITR Monoclonal Hybridoma Cells, and DNA Sequence Analysis of Monoclonal Hybridoma Antibodies

Subclone is intended to create monoclonal hybridoma cells to obtain monoclonal antibodies. The subclone method is based on a finite dilution procedure to obtain hybridoma cells producing a monoclone in a single well of a 96-well plate. After multiple rounds (3 rounds) of subclone, it was ensured that a resulting monoclone contained no other cellular components. For each round of subclone, ELISA, FACS and reporter gene activation activity assays were conducted. After three rounds of subclone, some of the monoclonal cells with high GITR binding and high stimulation activity were cryopreserved, and the rest were used for cell amplification culture to obtain sufficient monoclonal antibodies for purification and subsequent assays.

A total of 14 types of anti-GITR monoclonal hybridoma cells including 4E1, 8H4, 4E4, 3F5, 4C1, 7D7, 7E11, 10E11, 3G12, 4B11, 5F1, 5F6, 13G12 and 9B3 were cultured and antibody purified. Hybridoma cells were cultured in Dulbecco's Modified Eagle's medium (GIBCO; Invitrogen Corporation, Carlsbad, CA) containing 10% fetal bovine serum, 1% penicillin/streptomycin, 2% L-glutamine, and 1% NaHCOs solution for adjustion. The selected hybridoma cells were then adapted to a serum-free medium and antibodies were purified from the supernatant with Protein-G columns (GE Healthcare). After washing with PBS, bound antibodies were eluted with 0.1 M Glycine (pH 3.0), and then pH neutralized with 2.0 M Tris. Ultra-15 centrifugal concentrator (Amicon) was used for buffer exchange and antibody concentration.

### Example 4

Purified 4E1, 8H4, 4E4, 3F5, 4C1, 7D7, 7E11, 10E11, 3G12, 4B11, 5F1, 5F6, 13G12 and 9B3 hybridoma anti-hGITR monoclonal antibodies were further assayed for GITR binding, GITR ligand-blocking effect, GITR activation activity based on the reporter genes, and the property of cross-binding to monkey-derived GITR, and the assays involved ELISA, FACS and reporter gene activation analysis for Jurkat-GITR-NF-κB cells.

ELISA-based and FACS-based assays: the method was similar to the method used in Example 2, except that purified antibodies with determined concentrations were used in the assay, and EC50 and IC50 values were calculated with the antibody concentrations obtained by serial dilution and the corresponding results. Tables 4 to 7 show the assay results of 14 types of antibodies. In addition, the analysis of the property of cross-binding to monkey-derived GITR showed that there were cross-binding reactions between the selected 14 antibodies and monkey-derived GITR.

**Table 4 ELISA Binding-based EC50 of Hybridoma Anti-GITR Antibodies**

| ng/mL | 4E1 | 8H4 | 4E4 | 3F5 | 4C1 | 7D7 | 7E11 | 10E11 | 3G12 | 4B11 | 5F1 | 5F6 | 13G12 | 9B3 |
|---|---|---|---|---|---|---|---|---|---|---|---|---|---|---|
| EC50 | 0.067 | 1.338 | 1.705 | 2.314 | 1.932 | 1.037 | 1.144 | 4.733 | 1.628 | 0.883 | 2.039 | 1.259 | 0.277 | 0.523 |

**Table 5 FACS Binding-based EC50 of Hybridoma Anti-GITR Antibodies**

| ng/mL | 4E1 | 8H4 | 4E4 | 3F5 | 4C1 | 7D7 | 7E11 | 10E11 | 3G12 | 4B11 | 5F1 | 5F6 | 13G12 | 9B3 |
|---|---|---|---|---|---|---|---|---|---|---|---|---|---|---|
| EC50 | 0.416 | 20.41 | 19.92 | 6.026 | 8.3 | 6.387 | 7.844 | 9.173 | 33.06 | 15.67 | 51.74 | 29.35 | 77.31 | 1.923 |

**Table 6 Blocking Effect IC50 of Hybridoma Anti-GITR Antibodies on Binding of GITR-L to GITR Based on FACS Assay**

| ng/mL | 4E1 | 8H4 | 4E4 | 3F5 | 4C1 | 7D7 | 7E11 | 10E11 | 3G12 | 4B11 | 5F1 | 5F6 | 13G12 | 9B3 |
|---|---|---|---|---|---|---|---|---|---|---|---|---|---|---|
| EC50 | 12.91 | 457.6 | 112.7 | 906.2 | 97.29 | 102.3 | 70.82 | 89.77 | 322.0 | 318.5 | 1.513 | 118.2 | 4002 | 41.35 |

**Table 7 Stimulation Activity of Hybridoma Anti-GITR Antibodies in Jurkat-GITR-NF-κB Cell Reporter Gene Assay**

| Activity | 4E1 | 8H4 | 4E4 | 3F5 | 4C1 | 7D7 | 7E11 | 10E11 | 3G12 | 4B11 | 5F1 | 5F6 | 13G12 | 9B3 |
|---|---|---|---|---|---|---|---|---|---|---|---|---|---|---|
| Luc_signal | ++++ | ++ | +++ | +++ | +++ | +++ | +++ | +++ | +++ | +++ | +++ | +++ | ++ | +++ |

| | | | | | | | | | | | | | | |
|---|---|---|---|---|---|---|---|---|---|---|---|---|---|---|
| The number of + in the table represents the strength of the activity (a greater number of + means stronger activity). | | | | | | | | | | | | | | |

### Example 5: Assay of the Activity of Hybridoma GITR Antibodies in Stimulating Jurkat Cells to Release IL-2 Cytokines

To determine the immune activation activity of hybridoma anti-GITR antibodies, release of IL2 cytokines from Jurkat-GITR cells was used as one of the evaluation indicators of GITR signaling pathway activation. Jurkat-GITR cells were cultured in RPMI 1640 supplemented with 10% FBS. One day before culture, a 96-well plate was inoculated with goat anti-human IgG (Jackson, Cat No: 109-005-008) and goat anti-mouse IgG (Jackson, Cat No: 115-006-071) and left standing at room temperature overnight. After the wells of the plate were washed, 200 µL of blocking buffer was added, sealed, and incubated at 37°C for 1 hour. After the plate was washed 3 times with PBS, 40 ng/mL OKT3 (Ebioscience, Cat No: 16-0037-85) was added into the wells and incubated at 37°C for 1 hour. After the plate was washed again with PBS, 100 µL of Jurkat-GITR cells were added into each well of the 96-well plate (1×10⁵ cells per well) with or without diluted GITR antibodies. After incubation at 37°C for 48 or 72 hours, the 96-well plate was centrifuged and the supernatant was collected to measure IL-2 (R&D Systems, Cat No: DY202). The results are shown in Table 8.

**Table 8 Assay of the Activation Activity of Hybridoma Anti-GITR Antibodies on Immune Cells Based on IL-2 Release of Jurkat-GITR Cells**

| Activity | 4E1 | 8H4 | 4E4 | 3F5 | 4C1 | 7D7 | 7E11 | 10E11 | 3G12 | 4B11 | 5F1 | 5F6 | 13G12 | 9B3 |
|---|---|---|---|---|---|---|---|---|---|---|---|---|---|---|
| IL2 | ++ | +++ | ++++ | +++ | ++ | +++ | ++ | +++ | ++ | +++ | ++ | ++ | + | +++ |

| | | | | | | | | | | | | | | |
|---|---|---|---|---|---|---|---|---|---|---|---|---|---|---|
| The number of + in the table represents the strength of the activity (a greater number of + means stronger activity) | | | | | | | | | | | | | | |

### Example 6: Assay of Effects of Hybridoma Anti-GITR Antibodies on CD4 T Cell Activation and Cytokine Production

To further evaluate the activity of the hybridoma GITR antibodies in activating the GITR signaling pathway, primary lymphocyte effector cells were used in this study to assay the stimulation effects of the antibodies on IL2 and IFNγ cytokine release of immune cells. Human CD4 T cells were purified from human PBMCs using CD4 T cell negative selection purification kit and RPMI 1640 medium supplemented with 10% FBS. One day before the purification of T cells, a 96-well plate was inoculated with goat anti-human IgG (Jackson, Cat No: 109-005-008) and goat anti-mouse IgG (Jackson, Cat No: 115-006-071) and left standing at room temperature overnight. After the wells of the plate were washed, 200 µL of blocking buffer was added, sealed, and incubated at 37°C for 1 hour. After the plate was washed 3 times with PBS, 40 ng/mL OKT3 (Ebioscience, Cat No: 16-0037-85) was added into the wells and incubated at 37°C for 1 hour. After the plate was washed with PBS, 100 µL of CD4 T cells were added into each well of the 96-well plate (1×10⁵ cells per well) with or without diluted GITR antibodies. After incubation at 37°C for 48 or 72 hours, the 96-well plate was centrifuged, the supernatant was collected, and then ELISA kit (R&D Systems) was used to measure IL-2 (R&D Systems, Cat No: DY202) and IFN-γ (Cat No: DY285). The results are shown in Table 9.

**Table 9 Activation Activity of Hybridoma Anti-GITR Antibodies on Immune Cells Based on the Assay of IL-2 and IFNg Release of Primary CD4 T Cells.**

| Activity | 4E1 | 8H4 | 4E4 | 3F5 | 4C1 | 7D7 | 7E11 | 10E11 | 3G12 | 4B11 | 5F1 | 5F6 | 13G12 | 9B3 |
|---|---|---|---|---|---|---|---|---|---|---|---|---|---|---|
| IL2 | +++ | NA | +++ | NA | ++ | ++++ | ++ | +++ | ++ | ++++ | ++ | ++ | NA | ++++ |
| IFN-g | +++ | NA | ++++ | NA | +++/- | +++++ | +++ | +++ | +++ | ++++ | +++ | ++++/- | NA | +++ |

| | | | | | | | | | | | | | | |
|---|---|---|---|---|---|---|---|---|---|---|---|---|---|---|
| The number of + in the table represents the strength of the activity (a greater number of + means stronger activity) | | | | | | | | | | | | | | |

### Example 7: CDNA Clone and Sequence Analysis of GITR Hybridoma Monoclonal Antibodies

DNA sequencing was performed to the 14 anti-GITR monoclonal hybridoma antibodies 4E1, 8H4, 4E4, 3F5, 4C1, 7D7, 7E11, 10E11, 3G12, 4B11, 5F1, 5F6, 13G12 and 9B3. The corresponding amino acid sequence analysis results are shown in Tables 1 to 3.

The clonal analysis of the GITR antibody variable region genes included the following steps: the GITR monoclonal hybridoma cells were lysed with TRlzon (Cwbiotech, Cat No: CW0580), and the total RNA of the hybridoma cells was extracted. The RNA of the hybridoma cells was reversely transcribed into cDNA by using HiFi Script cDNA Synthesis Kit (Cwbiotech, Cat No: CW2569). The heavy chain and light chain variable region genes of the antibodies were amplified in a PCR method using the cDNA as a template, and degenerate primers (Kettleborough et al. (1993) Eur. J Immunology 23: 206-211; Strebe et al. (2010) Antibody Engineering 1:3-14). Reactions were performed in a S1000TM thermal cycler (Bio-Rad, CAT No: 184-2000) for 30 cycles: denaturation at 94°C for 1.5 minutes; annealing at 50°C for 1 minute; and synthesis at 72 °C for 1 minute. At the end of the 30^{th} cycle, the reacting mixture was incubated at 72°C for 7 more minutes for extension.

Electrophoresis of the PCR mixture was run on a 1% agarose/Tris-borate gel containing 0.5 µg/mL ethidium bromide. DNA fragments of an expected size (approximately 400 bp for heavy and light chains) were excised from the gel and purified. 3 µL of the purified PCR product was cloned into a pMD-18T vector (Takara, CAT No: D101A), to transform DH5a competent cells, and the DH5a competent cells were inoculated and cultured overnight at 37°C. Monoclones were picked from the culture plate and cultured for amplification, then plasmids were extracted, and the gene sequences of the antibodies were identified. Based on the gene sequences of the antibodies, the complementarity-determining regions (CDRs) and framework regions of the antibodies were analyzed.

The amino acid sequences of the CDR regions of the heavy chains and the light chains of the antibodies are shown in Table 1 and Table 2. The full-length amino acid sequences of the variable regions of the heavy chains and the light chains of the antibodies are shown in Table 3. The variable region sequences (see the table) of 14 antibodies 4E1, 8H4, 4E4, 3F5, 4C1, 7D7, 7E11, 10E11, 3G12, 4B11, 5F1, 5F6, 13G12 and 9B3 including respective light-chain variable sequences with SEQ ID NOs: 5, 13, 21, 29, 37, 45, 53, 61, 69, 77, 85, 93, 101 and 109, and respective heavy-chain variable sequences with SEQ ID NOs: 1, 9, 17, 25, 33, 41, 49, 57, 65, 73, 81, 89, 97 and 105, were amplified through respective hybridoma cloning. These antibodies showed some required functions, for example, GITR stimulation, increased T cell activation and cytokine release.

### Example 8: Humanization of GITR Monoclonal Hybridoma Antibodies

After comprehensive analysis and comparison (including those of the GITR-binding capacity, GITR ligand-blocking effect, GITR and immune cell activation activity, DNA sequencing of the antibodies, and the like) of the 14 anti-GITR monoclonal hybridoma antibodies 4E1, 8H4, 4E4, 3F5, 4C1, 7D7, 7E11, 10E11, 3G12, 4B11, 5F1, 5F6, 13G12 and 9B3, 4 anti-GITR monoclonal hybridoma antibodies 10E11, 4E4, 4B11 and 3F5 were selected for subsequent humanization transformation of GITR antibodies.

GITR antibodies were humanized in a complementarity-determining region grafting method. Firstly, the human germline antibody sequences with the maximum homology to the light chain and heavy chain variable regions of the murine-derived antibodies 10E11, 4E4, 4B11 and 3F5 were searched in the IMGT database. For the antibody 10E11 , germline IGKV3-15*01 was selected for the humanization of the light chain variable region, and IGHV5-10-1*01 was selected for the humanization of the heavy chain variable region. For the antibody 4E4, germline IGKV2-28*01 was selected for the humanization of the light chain variable region, and IGHV1-2*06 was selected for the humanization of the heavy chain variable region. For the antibody 4B11, IGKV3-11*01 was selected for the humanization of the light chain, and IGHV1-3*01 was selected for the humanization of the heavy chain. For the antibody 3F5, IGKV2-24*01 was selected for the humanization of the light chain, and IGHV1-2*02 was selected for the humanization of the heavy chain. The CDR regions of the murine-derived antibodies were retained, and the framework sequences of the murine-derived antibodies were replaced with the framework sequences of the human germline antibodies. Secondly, structural models of the murine-derived antibodies were established, and different amino acid sites between the structural models of the human antibodies and those of the corresponding murine-derived antibodies were compared one by one. If the spatial structure of the CDR region was not damaged or changed when a human amino acid sequence was used at a specific site in the framework, the human amino acid sequence would be used at the specific site, otherwise, the corresponding murine-derived sequence would be used at the site (that is, reverse mutation to the murine-derived sequence). Based on the structure simulation, some amino acids of the framework regions of the humanized antibodies 10E11, 4E4, 4B11 and 3F5 were reversely mutated to the murine-derived sequences.

Therefore, the CDR amino acid sequences of the human antibodies in the above template were replaced with the CDR amino acid sequences of the mouse antibodies 4E4, 10E11 , 4B11 and 3F5, respectively. In addition, the essential amino acid sequences of the VHs and VLs of the mouse antibodies 4E4, 10E11 , 4B11 and 3F5 were grafted into the frameworks of the VHs and VLs of the human antibodies in the above template, respectively, to obtain functionally humanized antibodies. In addition, for the VHs and VLs of 4E4, 10E11, 4B11 and 3F5, several sites of the framework amino acids of the human antibodies in the above template were reversely mutated to the corresponding amino acid sequences of the mouse antibodies 4E4, 10E11, 4B11 and 3F5.

For the humanized heavy chain of the antibody 10E11, Gly at the 24^{th} site was reversely mutated to Ala, Ser at the 28^{th} site was reversely mutated to Pro, Phe at the 29^{th} site was reversely mutated to Val, Thr at the 30^{th} site was reversely mutated to Ser, Met at the 48^{th} site was reversely mutated to Ile, lie at the 69^{th} site was reversely mutated to Phe, Lys at the 73^{rd} site was reversely mutated to Ile, and Ala at the 93^{rd} site was reversely mutated to Ser. For the humanized light chain of the antibody 10E11 , Tyr at the 49^{th} site was reversely mutated to Ser. For the humanized heavy chain of the antibody 4E4, Met at the 48^{th} site was reversely mutated to Ile, Val at the 67^{th} site was reversely mutated to Ala, Met at the 69^{th} site was reversely mutated to Leu, Arg at the 71^{th} site was reversely mutated to Val, and Thr at the 73^{th} site was reversely mutated to Lys. For the humanized light chain of the antibody 4E4, lie at the second site was reversely mutated to Val. For the humanized heavy chain of the antibody 4B11, Tyr at the 27^{th} site was reversely mutated to Phe, Met at the 48^{th} site was reversely mutated to Ile, Val at the 67^{th} site was reversely mutated to Ala, lie at the 69^{th} site was reversely mutated to Leu, Arg at the 71^{rd} site was reversely mutated to Ser, and Thr at the 73^{rd} site was reversely mutated to Lys. For the humanized light chain of the antibody 4B11, Tyr at the 49^{th} site was reversely mutated to Lys. Met at the 48^{th} site of a humanized heavy chain of the antibody 3F5 was reversely mutated to Ile, Val at the 67^{th} site was reversely mutated to Ala, Met at the 69^{th} site was reversely mutated to Leu, Arg at the 71^{th} site was reversely mutated to Val, and Thr at the 73^{th} site was reversely mutated to Lys. For the humanized light chain of the antibody 3F5, Leu at the 46^{th} site was reversely mutated to Arg.

The amino acid sequence numbers of the heavy chain and light chain variable regions of the humanized antibody 10E11 are SEQ ID NO: 119 and SEQ ID NO: 129, respectively. The amino acid sequence numbers of the heavy chain and light chain variable regions of the humanized antibody 4E4 are SEQ ID NO: 113 and SEQ ID NO: 114, respectively. The amino acid sequence numbers of the heavy chain and light chain variable regions of the humanized antibody 4B11 are SEQ ID NO: 115 and SEQ ID NO: 116, respectively. The amino acid sequence numbers of the heavy chain and light chain variable regions of the humanized antibody 3F5 are SEQ ID NO: 117 and SEQ ID NO: 118, respectively. The IgG1 subtypes of the humanized antibodies 10E11, 4E4, 4B11, and 3F5 were constructed; and human IgG1 (hIgG1) versions of the humanized antibodies 4E4, 10E11, and 4B11, a higG1 (3A) version of the humanized antibody 10E11, and hlgG4 versions of the humanized antibodies 10E11 and 3F5: h10E11-hlgG1, h4E4-hlgG1, h4B11-hlgG1, h10E11-hlgG1 (3A), h10E11-higG4, and h3F5-hlgG4, were produced. As shown in Table 3, the amino acid sequences include h4E4-IgG1 [SEQ ID NOs: 121 (full-length heavy chain) and 122 (full-length light chain)], h10E11-IgG1 [(SEQ ID NOs: 127 and 128 (full-length heavy chain) and 131 (full-length light chain))], h10E11-lgG1 (3A) [(SEQ ID NOs: 129 (full-length heavy chain) and 131 (full-length light chain))], h10E11-IgG4 [(SEQ ID NOs: 130 (full-length heavy chain) and 131 (full-length light chain))], h4B11-IgG1[(SEQ ID NOs: 123 (full-length heavy chain) and 124 (full-length light chain))] and h3F5-IgG1[(SEQ ID NOs: 125 (full-length heavy chain) and 126 (full-length light chain))].

Construction and expression of the humanized antibodies 4E4, 10E11, 4B11, and 3F5: the DNA encoding the light and heavy chains of the humanized antibodies was synthesized and cloned into expression vector pcDNA3.1 (Invitrogen, CAT No: #V-790). Freestyle 293 cells (200 mL, 10⁶/mL) were transfected with 100 µg of each type of the humanized heavy and light chain expression plasmid and cultured for 6 days. Then, the humanized antibodies were purified from the supernatant with Protein-G columns (GE Healthcare).

### Example 9

The binding of the humanized antibodies to GITR was determined via ELISA and FACS, the GITR ligand-blocking activity was determined via FACS, and the immune activation response of the humanized antibodies was analyzed based on the Jurkat-GITR-NF-κB cell reporter gene assay.

After production and purification of the humanized antibodies, the binding and specificity of the antibodies were determined via ELISA and FACS-based binding assays and assay of the blocking of the binding of Jurkat-GITR cells to GITR-L. The method used was similar to the methods described in Examples 2 and 4.

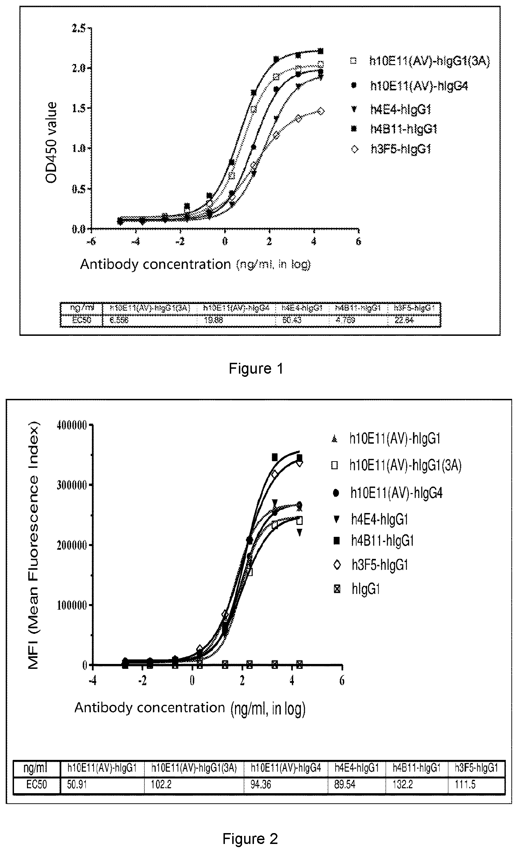

In the ELISA-based binding assay, it was shown that the humanized antibodies significantly bound to GITR, as shown in FIG. 1, the EC50 for the binding of the humanized anti-GITR antibodies to GITR-mFc protein was detected via ELISA; and the humanized anti-GITR antibodies included h10E11(AV)-hlgG1(3A), h10E11(AV)-hlgG4, h4E4-hlgG1, h4B11-hlgG1 and h3F5-hlgG1. An upper part of the figure shows the absorbance values of the humanized antibodies or the control hlgG1 having a series of concentrations. As indicated by the X axis, the concentrations of the test antibodies were 20000 ng/mL, 2000 ng/mL, 200 ng/mL, 20 ng/mL, 2 ng/mL, 0.2 ng/mL, 0.02 ng/mL, 0.002 ng/mL, 0.0002 ng/mL, and 0.00002 ng/mL. The table at the bottom of the figure shows the calculated EC50 for each type of the test antibodies. As shown in the figure, all the humanized antibodies significantly bound to GITR-mFc inoculated in plates, and h4B11-hlgG1 and h10E11(AV)-hlgG1 (3A) had stronger binding.

FIG. 2 shows the binding to GITR on Jurkat-GITR cells based on FACS. The bottom table provides the EC50 of each type of the humanized antibodies. 4B11-hlgG1 and 10E11(AV)-hlgG1 were the main conjugates based on FACS assay. The humanized GITR antibodies included h10E11(AV)-hlgG1, h10E11(AV)-hlgG1(3A), h10E11(AV)-hlgG4, h4E4-hlgG1, h4B11-hlgG1, h3F5-hlgG1 and a negative control (human IgG1). An upper part of the figure shows the mean fluorescence indexes of the humanized antibodies or the control hlgG1 having a series of concentrations. The bottom part shows the calculated EC50 of each type of the test antibodies. The results showed that all the humanized antibodies significantly bound to Jurkat-GITR cells. In addition, the humanized antibodies h4E4 and h10E11 (AV) were the main binders. As indicated by the X axis, the concentrations of the test antibodies were 20000 ng/mL, 2000 ng/mL, 200 ng/mL, 20 ng/mL, 2 ng/mL, 0.2 ng/mL, 0.02 ng/mL, and 0.002 ng/mL, respectively.

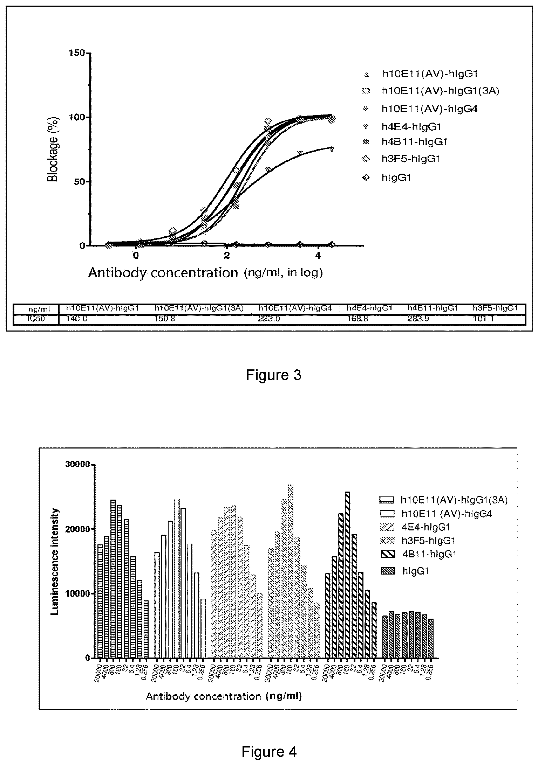

FIG. 3 shows the blocking of the binding of GITR-L to GITR by the humanized antibodies on Jurkat-GITR cells. The concentration of the GITR-L used was 0.25 µg/mL. The test GITR antibodies included h10E11 (AV)-hlgG1, h10E11 (AV)-hIgG1(3A), h10E11 (AV)-hlgG4, h4E4-hlgG1, h4B11-hIgG1, h3F5-hlgG1 and a negative control (human IgG1). As indicated by the X axis, the concentrations of the test antibodies were 20000 ng/mL, 4000 ng/mL, 800 ng/mL, 160 ng/mL, 32 ng/mL, 6.4 ng/mL, 1.28 ng/mL, and 0.256 ng/mL, respectively. The results showed that all of the humanized GITR antibodies showed certain blocking activity on the binding of GITR-L to Jurkat-GITR cells. The humanized antibodies h10E11 (AV) and h3F5 had better blocking effects.

FIG. 4 shows the GITR activation activity of the humanized anti-GITR antibodies via 293T-GITR-NFkb-luc cell reporter gene assay. The test anti-GITR antibodies included h10E11(AV)-hIgG1(3A), h10E11(AV)-hlgG1, h4E4-hlgG1, h4B11-hlgG1, h3F5-hlgG1 and a negative control (human lgG1). The concentrations of the test antibodies were 20000 ng/mL, 4000 ng/mL, 800 ng/mL, 160 ng/mL, 32 ng/mL, 6.4 ng/mL, 1.28 ng/mL, and 0.256 ng/mL, respectively. The results showed that all of the humanized antibodies had strong activation activity for the expression of the luciferase reporter gene in 293T-GITR-NFkb-luc cells. Similar activity was observed among the test humanized antibodies.

### Example 10: Activation Test of the Humanized Anti-GITR Antibodies via Jurkat-GITR IL-2 Release Assay

To test the activation activity of the hybridoma anti-GITR antibodies, the antibodies were first evaluated by their stimulation effects on GITR signaling pathway in Jurkat-GITR cells. Jurkat-GITR, 293T-OKT3, 293T-CD32A and 293T cells were each cultured in RPMI 1640 supplemented with 10% FBS. 50 µL of Jurkat-GITR (1×10⁵ cells per well), 50 µL of 293T-OKT3 (2×10⁴ cells per well), and 50 µL of 293T-CD32A or 293T (2×10⁴ cells per well) cells were added into individual wells of a 96-well plate. It was determined whether there were diluted GITR antibodies. After incubation at 37°C for 48 or 72 hours, the 96-well plate was centrifuged and the supernatant was collected to measure IL-2 (R&D Systems, Cat No: DY202).

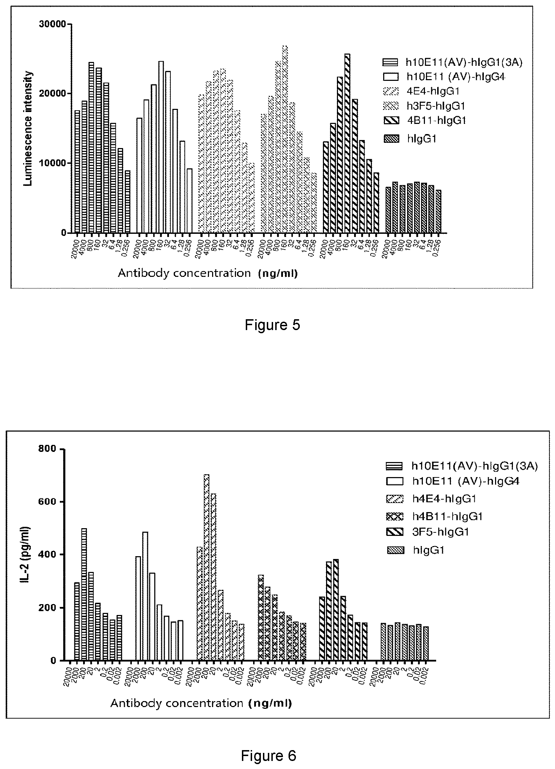

FIG. 5 shows the result of the activation of Jurkat-GITR cells by the humanized GITR antibodies to release cytokine IL2 without cross-linking. Jurkat-GITR cells were stimulated by OKT3 in 293T-OKT3 cells to some extent. The test anti-GITR antibodies included h10E11 (AV)-hlgG1(3A), h10E11(AV)-hlgG1, h4E4-hlgG1, h4B11-hlgG1, h3F5-hlgG1 and a negative control (human lgG1). The concentrations of the test antibodies were 20000 ng/mL, 4000 ng/mL, 800 ng/mL, 160 ng/mL, 32 ng/mL, 6.4 ng/mL, 1.28 ng/mL, and 0.256 ng/mL, respectively. As shown in the figure, all the humanized antibodies can stimulate, to some extent, Jurkat-GITR cells to release IL2 without cross-linking.

FIG. 6 shows the result of the activation of Jurkat-GITR cells by the humanized GITR antibodies to release cytokine IL2 with cross-linking. The cross-linking factor is CD32a expressed in 293T-CD32a cells. Jurkat-GITR cells were stimulated by OKT3 in 293T-OKT3 cells to some extent. The test anti-GITR antibodies included h10E11(AV)-hIgG1(3A), h10E11(AV)-hIgG1, h4E4-hlgG1, h4B11-hlgG1, h3F5-hlgG1 and a negative control (human lgG1). The concentrations of the test antibodies were 20000 ng/mL, 4000 ng/mL, 800 ng/mL, 160 ng/mL, 32 ng/mL, 6.4 ng/mL, 1.28 ng/mL, and 0.256 ng/mL, respectively. As shown in the figure, all the humanized antibodies exhibited stronger stimulation activity on IL2 release from Jurkat-GITR cells (especially h4E4-hlgG1) with cross-linking.

### Example 11: Effect of Humanized Anti-GITR Antibodies on Cytokine Production by CD4+ T Cells

The activity of the GITR hybridoma antibodies was evaluated by their stimulation effect on GITR signaling pathway in lymphocyte effector cells. Human CD4+ T cells were purified from PBMCs by using CD4+ negative selection kit in RPMI 1640 supplemented with 10% FBS. One day before culture, a 96-well plate was inoculated with goat anti-human IgG (Jackson, Cat No: 109-005-008) and goat anti-mouse IgG (Jackson, Cat No: 115-006-071) and left standing at room temperature overnight. After the wells were washed, 200 µL of blocking buffer was added, sealed, and incubated at 37°C for 1 hour. After the plate was washed 3 times with PBS, 40 ng/mL OKT3 (Ebioscience, Cat No: 16-0037-85) was added into the wells and incubated at 37°C for 1 hour. After the plate was washed with PBS, 100 µL of CD4+ T cells were added into each well of the 96-well plate (1×10⁵ cells per well) with or without diluted GITR antibodies. After incubation at 37°C for 48 or 72 hours, the 96-well plate was centrifuged, the supernatant was collected, and ELISA kit (R&D Systems) was used to measure IL-2 (R&D Systems, Cat No: DY202) and IFN-γ (Cat No: DY285).

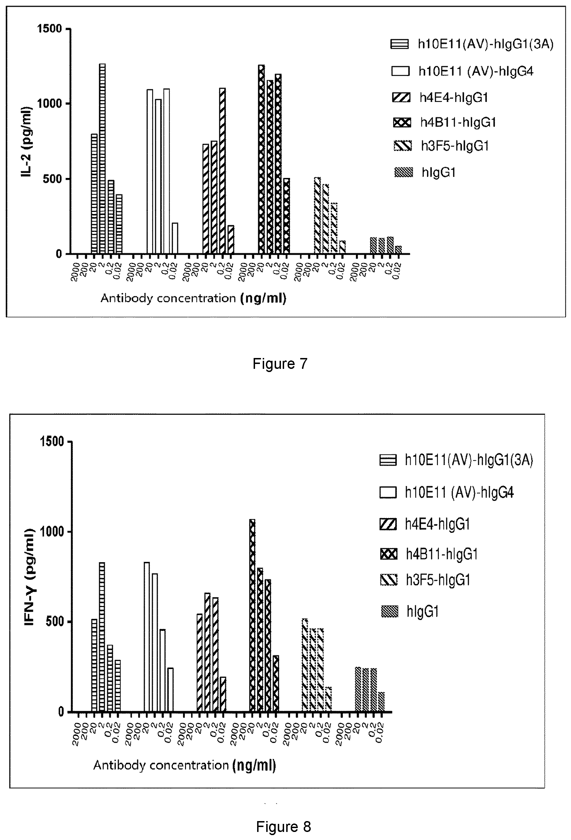

FIG. 7 shows the effect of the humanized GITR antibodies on the production of cytokine IL2 by CD4+ T cells. The test anti-GITR antibodies included h10E11(AV)-hIgG1(3A), h10E11(AV)-hlgG1, h4E4-hlgG1, h4B11-hlgG1, h3F5-hlgG1 and a negative control (human lgG1). The concentrations of the test antibodies were 20 ng/mL, 2 ng/mL, 0.2 ng/mL, and 0.02 ng/mL, respectively. As shown in the figure, the humanized antibodies 10E11, 4B11 and 4E4 (in particular, h4B11-hIgG1) had significant stimulation effects on the cytokine IL2 production by the primary CD4 T cells; and the humanized 3F5 antibody in this assay showed relatively low activity.

FIG. 8 shows the effect of the humanized GITR antibodies on the production of cytokine IFN-γ by CD4+ T cells. The test anti-GITR antibodies included h10E11 (AV)-hlgG1 (3A), h10E11(AV)-hlgG1, h4E4-hlgG1, h4B11-hlgG1, h3F5-hlgG1 and a negative control (human lgG1). The concentrations of the antibodies were 20 ng/mL, 2 ng/mL, 0.2 ng/mL, and 0.02 ng/mL, respectively. As shown in the figure, the humanized antibodies 10E11, 4B11 and 4E4 (in particular, h4B11-hIgG1) had significant stimulation effects on the cytokine IFN-γ production of the primary CD4 T cells; and the humanized 3F5 antibody in this assay showed relatively low activity. The results indicated a similar pattern to that of IL2 production in FIG. 7. That is, the humanized antibodies 10E11, 4B11 and 4E4 had significant stimulation effects on the IL2 production of the primary CD4 T cells. The humanized 3F5 antibody showed relatively low activation activity.

### Example 12: Biacore Kinetics Test of Humanized Anti-GITR Antibodies 4E4, 10E11, 4B11 and 3F5

To characterize the binding properties of the humanized antibodies, the binding kinetics between GITR and anti-GITR antibodies was measured with Biacore 3000 and recorded at a data collection rate of 1 Hz. Polyclonal rabbit anti-mouse IgG (GE, BR-1008-38) was diluted with 10 mM sodium acetate (pH 5.0) and immobilized in reference and test flow cells of a CM5 biosensor chip to about 15000RU by using an amine coupling kit (GE, BR10050). At the beginning of each cycle, diluted test antibodies (1.5 µg/mL) were injected into the test flow cell for 1 minute to be captured. Preparation method of GITR analyte series included: a stock solution was diluted to have a concentration of 100 nM with a running buffer, followed by 2-fold serial dilutions to have a concentration of 0.78 nM with the same buffer. The analytes were continuously injected into the reference and test flow cells at a flow rate of 30 µL/min for 3 minutes. Then, a running buffer (PBS containing 0.05% P20) was injected at a flow rate of 30 µL/min for 10 minutes. At the end of each cycle, 10 mM glycine-HCl buffer (pH 1.7) was injected at a flow rate of 10 µL/min for 3 minutes for regeneration on the biosensor surface. For each injection of the analyte sample (that is, each cycle), dual reference was performed to the binding responses obtained on a test biosensor surface by subtracting simultaneously recorded responses from a reference surface and subtracting responses from a single reference running buffer sample. Association and dissociation rate constants (ka and kd) may be both determined by fitting dual-reference sensorgrams of the entire titration series to Langmuir model (1:1) with Biaevaluation 4.0 software. A dissociation constant KD is calculated by using the determined rate constant via a formula KD=kd/ka. As shown in Table 10, the humanized anti-GITR antibodies bound to human GITR with high affinity, especially humanized 4B11 and 10E11.

**Table 10 GITR-his multi-cycle kinetics test by SPR**

| Antibody | Ka (1/MS) | Kd (1/S) | KD (M) |
|---|---|---|---|
| Humanized 10E11 | 4.24E+05 | 2.93E-04 | 6.91E-10 |
| Humanized 4E4 | 3.25E+05 | 1.23E-03 | 3.78E-09 |
| Humanized 4B11 | 1.73E+06 | 2.57E-04 | 1.49E-10 |

| | | | |
|---|---|---|---|
| Biacore T200 test; Model: kinetics, 1:1 binding; KD=kd/ka | | | |

The results of the foregoing examples show that the monoclonal antibody or the antigen-binding fragment thereof according to the present disclosure, or the monoclonal antibody conjugate comprising the monoclonal antibody or the antigen-binding fragment thereof according to the present disclosure has good application potential in preparation of medications for activating GITR signaling, medications for activating T lymphocytes, medications for increasing the expression of IL-2 and INF-γ in T lymphocytes, medications for blocking the binding of GITR-L to GITR, and medications for preventing, treating, and/or assistantly treating tumors.

The foregoing embodiments are only preferable embodiments of the present disclosure. It should be noted that a person of ordinary skills in the art could also make changes without departing from the principle of the present disclosure. These changes shall also be construed as falling within the protection scope of the present disclosure.

## Claims

1. An anti-GITR monoclonal antibody or an antigen-binding fragment thereof, which comprises a heavy chain and a light chain, wherein an amino acid sequence of CDR1 of the heavy chain is selected from the group consisting of SEQ ID NOs: 2, 10, 18, 26, 34, 42, 50, 58, 66, 74, 82, 90, 98 and 106; an amino acid sequence of CDR2 of the heavy chain is selected from the group consisting of SEQ ID NOs: 3, 11, 19, 27, 35, 43, 51, 59, 67, 75, 83, 91, 99 and 107; an amino acid sequence of CDR3 of the heavy chain is selected from the group consisting of SEQ ID NOs: 4, 12, 20, 28, 36, 44, 52, 60, 68, 76, 84, 92, 100 and 108; an amino acid sequence of CDR1 of the light chain is selected from the group consisting of SEQ ID NOs: 6, 14, 22, 30, 38, 46, 54, 62, 70, 78, 86, 94, 102 and 110; an amino acid sequence of CDR2 of the light chain is selected from the group consisting of SEQ ID NOs: 7, 15, 23, 31, 39, 47, 55, 63, 71, 79, 87, 95, 103 and 111; and an amino acid sequence of CDR3 of the light chain is selected from the group consisting of SEQ ID NOs: 8, 16, 24, 32, 40, 48, 56, 64, 72, 80, 88, 96, 104 and 112, and wherein the heavy and light chains of the antigen-binding fragment comprise amino acid sequences spanning from CDR1 through CDR3 of the heavy and light chains of the antibody, respectively.

2. The anti-GITR monoclonal antibody or the antigen-binding fragment thereof according to claim 1, wherein an amino acid sequence of a heavy chain variable region is selected from the group consisting of SEQ ID NOs: 1, 9, 17, 25, 33, 41, 49, 57, 65, 73, 81, 89, 97 and 105, and an amino acid sequence of a light chain variable region is selected from the group consisting of SEQ ID NOs: 5, 13, 21, 29, 37, 45, 53, 61, 69, 77, 85, 93, 101 and 109.

3. The anti-GITR monoclonal antibody or the antigen-binding fragment thereof according to claim 1, wherein the heavy chain and the light chain are humanized; and an amino acid sequence of a humanized heavy chain variable region is selected from the group consisting of SEQ ID NOs: 113, 115, 117 and 119; and an amino acid sequence of a humanized light chain variable region is selected from the group consisting of SEQ ID NOs: 114, 116, 118 and 120.

4. The anti-GITR monoclonal antibody or the antigen-binding fragment thereof according to claim 3, wherein a full-length amino acid sequence of the humanized heavy chain is selected from the group consisting of SEQ ID NOs: 121, 123, 125, 127, 128, 129 and 130, and a full-length amino acid sequence of the humanized light chain is selected from the group consisting of SEQ ID NOs: 122, 124, 126 and 131.

5. A nucleotide sequence encoding the anti-GITR monoclonal antibody or the antigen-binding fragment thereof according to any one of claims 1 to 4.

6. The anti-GITR monoclonal antibody or the antigen-binding fragment thereof according to any one of claims 1 to 4 for use in preparation of a medication, wherein the medication comprises but is not limited to a medication for blocking binding of GITR-L to GITR, a medication for activating T lymphocytes, or a medication for increasing expression of IL-2 and IFN- in T lymphocytes.

7. A monoclonal antibody conjugate, comprising a monoclonal antibody and a conjugating part, wherein the monoclonal antibody is the anti-GITR monoclonal antibody or the antigen-binding fragment thereof according to any one of claims 1 to 4, and the conjugating part is selected from one or more of a radionuclide, a medication, a toxin, a cytokine, a cytokine receptor fragment, an enzyme, fluorescein, and biotin.

8. The monoclonal antibody conjugate according to claim 7 for use in preparation of a medication, wherein the medication comprises but is not limited to a medication for blocking binding of GITR-L to GITR, a medication for activating T lymphocytes, or a medication for increasing expression of IL-2 and IFN- in T lymphocytes.

9. The monoclonal antibody conjugate according to claim 7 for use in preparation of a medication for preventing, treating, and/or assistantly treating a tumor.
